# EUROPÄISCHE PATENTSCHRIFT

(11) **EP 0 462 463 B1**
(45) Veröffentlichungstag und Bekanntmachung des Hinweises auf die Patenterteilung: **22.11.1995**
(21) Anmeldenummer: 91109373.0
(22) Anmeldetag: 07.06.1991
(51) Int. Cl.: G01N 33/80, G01N 33/555, B01L 3/00, B01L 3/14, B04B 7/00

(54) **Verfahren zur Bestimmung von Blutgruppen**
Method for the detection of blood groups
Procédé pour la détection des groupes sanguins

(30) Priorität: 16.06.1990 DE 4019299
(43) Veröffentlichungstag der Anmeldung: 27.12.1991
(73) Patentinhaber: Ballies, Uwe, Dr. med., 24103 Kiel (DE)
(72) Erfinder: Ballies, Uwe, Dr. med., 24103 Kiel (DE)
(74) Vertreter: Tönnies, Jan G., Dipl.-Ing.

(56) Entgegenhaltungen:
- EP-A- 0 050 018
- EP-A- 0 054 087
- DE-A- 3 038 210
- DE-A- 3 912 901

## Beschreibung

Die Erfindung betrifft ein Verfahren nach dem Oberbegriff des Anspruchs 1 und eine Vorrichtung zur Durchführung dieses Verfahrens.

Aus der DE 30 38 210 C2 sind ein Verfahren und eine Vorrichtung bekannt, bei denen eine immunologische Agglutinationsreaktion dadurch gemessen wird, daß die Probe durch Zentrifugation mit der reaktionsflüssigkeit in Kontakt gebracht wird und das sich bei Beendigung der Zentrifugation am Reaktionsgefäß bildende Agglutinationsmuster gelesen wird.

Ein anderes Verfahren zur Bestimmung der Agglutinationsreaktion durch Zentrifugieren wird in der EP 0 054 087 beschrieben.

Bei den vorbekannten Verfahren ist der apparative Aufwand erheblich, eine einfache Dokumentation des Ergebnisses ist nicht möglich.

Der Erfindung liegt nun die Aufgabe zugrunde, ein vereinfachtes, schnelleres, leichter handzuhabendes und eine leichtere Dokumentation erlaubendes Verfahren zu schaffen.

Die gestellte Aufgabe wird erfindungsgemäß durch ein Verfahren mit den Merkmalen des Anspruchs 1 gelöst. Gegenstand der Ansprüche 2 - 10 ist eine Vorrichtung zur Durchführung dieses Verfahrens.

Bei dem Vorschlag nach der Erfindung ist der nach der Agglutination zurückgelegte Weg ein Maß für die Bestimmung der Blutgruppe und den anderen gewünschten Informationen (je nach verwendetem Antiserum).

Durch diese Lösung der Aufgabe ist es möglich, wesentlich billigere Reaktionskammern, nämlich kleine Plättchen mit Vertiefungen die mit einem Deckel versehen werden, herzustellen, und es ist weiter möglich, auf das Gel zu verzichten. Ein besonderer Vorteil ergibt sich auch daraus, daß die kleinen Plättchen direkt zu Dokumentationszwecken archiviert werden können, sie verblassen nicht und das festgestellte Ergebnis bleibt stabil.

Weiter wird vorgeschlagen, die Engstelle oder die Engstellen zum Rand hin kontinuierlich flacher werden zu lassen. Alternativ oder additiv kann auch die Oberflächenrauhigkeit der Plättchen so verändert werden, daß dieser Effekt eintritt, bzw. kann eine stufenförmige Verflachung vorgenommen werden.

Vorteilhaft ist auch die Aufbringung verschiedener Indikatoren an verschiedenen oder hintereinander gelagerten Stellen der Verengungen. Bezüglich der Vertiefung in die das Blut eingegeben wird, wird vorgeschlagen, ein Loch in der Mitte des Plättchens zur Halterung in der Zentrifuge vorzusehen und einen Ringkanal um dieses Loch herum vorzusehen. Eine Sektorierung zur definierten Unterteilung dieses Kanals ist vorzuziehen.

Weiter ist es eine vorteilhafte Ausgestaltung, wenn eine erste Kapillarzone dem Ort des Serumeintrages direkt folgt.

In der Zeichnung sind mehrere Ausführungsbeispiele der Vorrichtung zum Bestimmen der Blutgruppe einer bestimmten Probe dargestellt. Es zeigt:

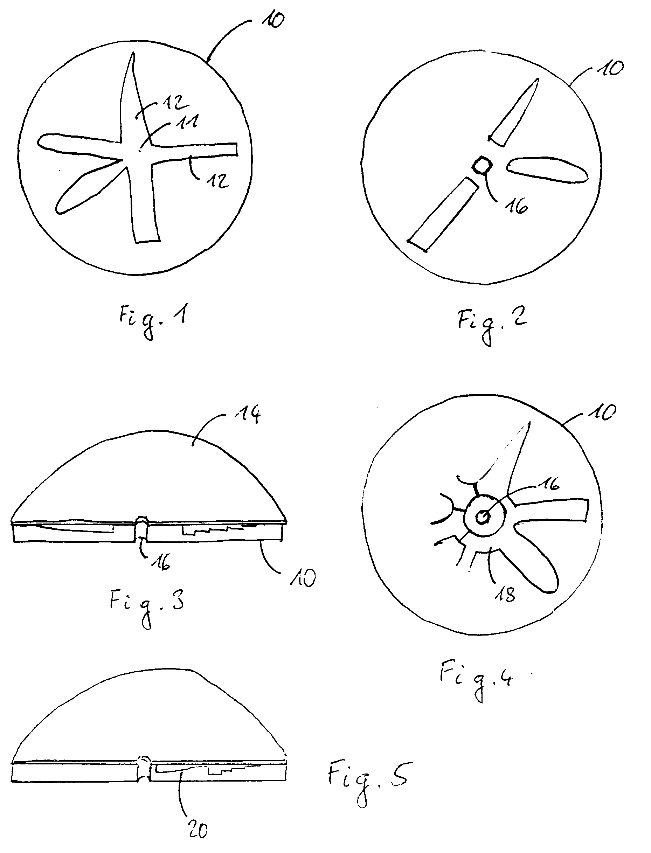

- Fig. 1: ein Plättchen ohne mittlere Ausnehmung mit einer zentralen Vertiefung zum Eingeben des zu prüfenden Mediums, mit fünf sich zum Rand hin erstreckenden verschieden geformten Vertiefungen,
- Fig. 2: eine Vorrichtung wie in Fig. 1, jedoch mit einer mittigen Ausnehmung und den Vertiefungen, die zum Rand hin flacher werden,
- Fig. 3: eine Vorrichtung wie in Fig. 2, in Schnittdarstellung mit einer stufenlos verflachenden Vertiefung, einer gestuft sich abflachenden Vertiefung und aufliegendem Deckplättchen,
- Fig. 4: eine Vorrichtung wie in Fig. 2, jedoch mit einem um die zentrale Ausnehmung verlaufenden Ringkanal, in dem auf der einen Seite eine Sektorierung angedeutet ist, und
- Fig. 5: eine Vorrichtung dargestellt wie in Fig. 3, jedoch mit einer ersten Kapillarzone.

Die Vorrichtung zur Durchführung der Blutgruppenbestimmung einer bestimmten Probe besteht aus einem unteren Scheibchen 10, das im Spritzguß hergestellt wird und mindestens eine Vertiefung 11 zur Aufnahme der zu untersuchenden Probe, insbesondere einer Erythrozytensuspension, und mindestens einer zusammen mit dem Deckplättchen 14 einen Engpaß darstellenden sich zum Rand hin erstreckenden Vertiefung 12.

Auf das Plättchen 10 und/oder das Deckplättchen 14 wird mindestens ein jedoch vorteilhafterweise mehrere jeweils für eine Blutgruppe spezielle Antiserum aufgebracht. Diese Seren sind lange haltbar. Wenn nun eine Probe auf das Plättchen 10 gebracht wird, wird das Serum zu einer Agglutination der Erythrozyten führen. Anschließend wird das Plättchen 10 in eine Zentrifuge eingebracht, die bei dieser Erfindung besonders kleine Ausmaße haben kann. Durch die Zentrifugalkraft wird das zur Probe eingebrachte Medium nach außen hin beschleunigt. Dabei versucht es entlang der Vertiefung 12 im Plättchens 10, die jetzt zusammen mit dem Deckplättchen eine Verengung darstellt, entlangzuwandern. Die Rauhigkeit der Wände sowie die optionale Verengung werden dazu führen, daß die agglutinierten Partikel früher sich nicht mehr fortbewegen können, als die nicht agglutinierten Erythrozyten.

Der Weg den agglutinierte bzw. nicht agglutinierte Partikel zurückgelegt haben, ist ein Maß dafür, in wie weit das Serum zur Agglutination führt und ist damit ein Maß, ob eine positive bzw. eine negative Reaktion auf dieses Serum stattfindet.

Nachdem die Plättchen 10 aus der Zentrifuge genommen worden sind werden die eingebrachten Medien langsam eintrocknen, nicht jedoch ihre Farbe verlieren. Dadurch ist eine Archivierung zu Dokumentationszwecken der verwendeten Plättchen direkt möglich. Nach den weiter vorgeschlagenen Ausführungsbeispielen kann das Einbringen in die Zentrifuge durch eine mittig angebrachte Ausnehmung 16 erleichtert werden. Diese Ausnehmung ist vorzugsweise nicht rund. Dann ist es vorteilhaft einen Ringkanal 18 um diese Ausnehmung vorzusehen.

Eine Sektorierung in diesem Kanal kann Vorteile bringen, sollte aber dann günstigerweise so vorgesehen werden, daß ein gleichmäßiger Serumeintrag gewährleistet ist, könnte also z.B. am Abdeckplättchen 14 vorgesehen sein.

Auf dem Plättchen 10 oder dem Deckplättchen 14 sollte ferner ein Bereich zur Dokumentation freibleiben, auf dem die Probe entsprechend markiert werden kann.

Das in Fig. 5 dargestellte Plättchen 10 besitzt zusätzlich noch eine erste Kapillarzone 20, an deren Wänden sich bereits die Antiseren befinden, und die es erlaubt, ohne Zentrifugieren schon eine Vorabinformation über die Agglutination des Serums zu erhalten.

## Patentansprüche

1. Verfahren zur Bestimmung der Blutgruppe einer bestimmten Probe durch Messen der Erythrozyten-Agglutinationsreaktion, bei dem die Erythrozyten der Probe mit für eine Blutgruppe speziellen Antiseren in Kontakt gebracht und zentrifugiert werden,
dadurch gekennzeichnet, daß
die mit den Antiseren in Kontakt gekommenen Erythrozyten durch das Zentrifugieren durch benachbart angeordnete Wände definierte, mechanisch fest vorgegebene Engstellen geführt werden.

2. Vorrichtung zur Durchführung des Verfahrens in Anspruch 1, gekennzeichnet durch eine kreisrunde Scheibe (10) mit mehreren radial verlaufenden Vertiefungen (12), wobei in jede der Vertiefungen wenigstens ein Antiserum eingebracht ist und die Vertiefungen durch ein Plättchen (14) abgedeckt sind.

3. Vorrichtung nach Anspruch 2, dadurch gekennzeichnet, daß die eine oder mehreren Vertiefungen (12) nach außen flacher werden.

4. Vorrichtung nach Anspruch 2 oder 3, dadurch gekennzeichnet, daß das Material der kreisförmigen Scheibe eine besondere Oberflächenrauhigkeit hat.

5. Vorrichtung nach einem der vorangehenden Ansprüche, dadurch gekennzeichnet, daß die Tiefe der Vertiefungen zum Rand hin stufenförmig abnimmt.

6. Vorrichtung nach einem der vorangehenden Ansprüche, dadurch gekennzeichnet, daß verschiedene Indikatoren an verschiedenen Stellen der Vertiefungen aufgebracht sind.

7. Vorrichtung nach einem der vorangehenden Ansprüche, gekennzeichnet durch ein zentrales, der Befestigung dienendes Loch (16) im Plättchen vorgesehen ist.

8. Vorrichtung nach einem der vorangehenden Ansprüche, gekennzeichnet durch einen das zentrale Loch (16) umgebenden Ringkanal (18) zum Einbringen der Probe.

9. Vorrichtung nach einem der vorangehenden Ansprüche, dadurch gekennzeichnet, daß die das eingebrachte Serum aufnehmende mittige Vertiefung in Sektoren unterteilt ist.

10. Vorrichtung nach einem der vorangegangenen Ansprüche, gekennzeichnet durch eine erste Kapillarzone (20).

## Claims

1. Process for the determination of the blood group of a particular sample by measuring the erythrocyte agglutination reaction, in which the erythrocytes of the sample are contacted with anti-sera specific to a blood group and are centrifuged, characterized in that the erythrocytes brought into contact with the antisera as a result of centrifuging are guided by neighbouring walls to defined, mechanically predetermined narrow points.

2. Apparatus for performing the process according to claim 1, characterized by a circular disk (10) with several radially directed depressions (12) and into each of the latter is introduced at least one antiserum and the depressions are covered by a platelet (14).

3. Apparatus according to claim 2, characterized in that one or more depressions (12) become shallower to the outside.

4. Apparatus according to claim 2 or 3, characterized in that the material of the circular disk has a particular surface roughness.

5. Apparatus according to one of the preceding claims, characterized in that the depth of the depressions decreases in step-like manner towards the edge.

6. Apparatus according to one of the preceding claims, characterized in that different indicators are applied to different points of the depressions.

7. Apparatus according to one of the preceding claims, characterized by a central hole (16) in the platelet used for fixing purposes.

8. Apparatus according to one of the preceding claims, characterized by a ring duct (18) surrounding the central hole (16) for introducing the sample.

9. Apparatus according to one of the preceding claims, characterized in that the central depression receiving the introduced serum is subdivided into sectors.

10. Apparatus according to one of the preceding claims, characterized by a first capillary zone (20).

## Revendications

1. Procédé de détermination du groupe sanguin d'un échantillon déterminé, par mesure de la réaction d'agglutination d'érythrocyte, dans laquelle les érythrocytes de l'échantillon sont mis en contact avec des anti-sérums spéciaux pour un groupe sanguin et sont soumis à une centrifugation caractérisé en ce que les érythrocytes venant en contact avec les anti-sérums sont guidés sous l'effet de la centrifugation à travers des points de rétrécissement prédéterminés, mécaniquement fixés, définis par des parois placées voisines les unes des autres.

2. Dispositif de mise en oeuvre du procédé selon la revendication 1, caractérisé par un disque (10) circulaire, comportant plusieurs cavités (12) s'étendant radialement, au moins un anti-sérum étant introduit dans chacune des cavités et les cavités étant recouvertes d'une plaquette (14).

3. Dispositif selon la revendication 2, caractérisé en ce que les cavités (12), qu'elles soient au nombre de un ou plus, s'aplatissent en direction de l'extérieur.

4. Dispositif selon la revendication 2 ou 3, caractérisé en ce que le matériau du disque circulaire a une rugosité de surface particulière.

5. Dispositif selon l'une des revendications précédentes, caractérisé en ce que la profondeur des cavités diminue par degrés vers le bord.

6. Dispositif selon l'une des revendications précédentes, caractérisé en ce que différents indicateurs sont appliqués en différents endroits des cavités.

7. Dispositif selon l'une des revendications précédentes, caractérisé par un trou central (16) servant à la fixation, prévu dans la plaquette.

8. Dispositif selon l'une des revendications précédentes, caractérisé par un canal annulaire (18) entourant le trou central (16), en vue d'introduire l'échantillon.

9. Dispositif selon l'une des revendications précédentes, caractérisé en ce que la cavité centrale recevant le sérum introduit est subdivisée en secteurs.

10. Dispositif selon l'une des revendications précédentes, caractérisé par une première zone capillaire (20).
